# EUROPÄISCHE PATENTSCHRIFT

(11) **EP 2 364 644 B1**
(45) Veröffentlichungstag und Bekanntmachung des Hinweises auf die Patenterteilung: **13.04.2016**
(21) Anmeldenummer: 11155181.8
(22) Anmeldetag: 21.02.2011
(51) Int. Cl.: A61B 5/053

(54) **Elektromedizinisches Implantat und Überwachungssystem**
Electromedical implant and monitoring system
Implant électromédical et système de surveillance

(30) Priorität: 09.03.2010 US 311792 P
(43) Veröffentlichungstag der Anmeldung: 14.09.2011
(73) Patentinhaber: BIOTRONIK SE & Co. KG, 12359 Berlin (DE)
(72) Erfinder: Skerl, Olaf, 18209 Bad Doberan (DE); Lippert, Michael, 91522 Ansbach (DE); Czygan, Gerald, 91054 Buckenhof (DE)
(74) Vertreter: Galander, Marcus

(56) Entgegenhaltungen:
- WO-A1-2004/047638
- WO-A1-2009/035596
- WO-A2-2005/010640

## Beschreibung

Die Erfindung betrifft ein elektromedizinisches Implantat zum Überwachen einer thorakalen Eigenschaft eines Lebewesens, wobei das Implantat ein Gehäuse hat. Weiter betrifft die Erfindung ein Überwachungssystem mit dem elektromedizinischen Implantat.

Ein elektromedizinisches Implantat der eingangs genannten Art kann ein reines Monitoringimplantat oder ein Monitoring- und Therapieimplantat sein. Als sogenanntes Monitoringimplantat - im Unterschied zu Therapieimplantaten, wie implantierbare Herzstimulatoren oder dergleichen - hat ein elektromedizinisches Implantat zum Überwachen einer thorakalen Eigenschaft eines Lebewesens (Patienten) keine Therapieoptionen. Ein Monitoringimplantat kann auch für Patienten ohne Therapieindikation sinnvoll und wichtig sein, um durch Überwachen einer thorakalen Eigenschaft des Patienten Rückschlüsse auf den Gesundheitszustand zu ziehen. So kann sich beispielsweise erst aus einer längerfristigen Überwachung des Patienten mit dem Monitoringimplantat eine Indikation zur Elektrotherapie ergeben bzw. eine Art der Elektrotherapie im Vorfeld derselben genauer spezifizieren lassen.

Bisherige Ansätze zum Überwachen einer thorakalen Eigenschaft, nämlich der Pulswellenlaufzeit, eines Lebewesens nutzen weitgehend nicht-invasive Systeme, wie sie beispielsweise in US 7,029,447 oder US 6,748,262 oder US 5,743,856 zur Überwachung kardialer oder epithorakaler, peripherer Blutströme beschrieben sind. Nicht-invasive Systeme sind im Zweifel jedoch umständlich in der Handhabung, wenn es darum geht, eine thorakale Eigenschaft eines Lebewesens über einen längeren Zeitraum zu überwachen. Andererseits ist ein kardial invasives System wie das in DE 36 29 587 A1 keine Option für Patienten ohne Therapieindikation. Die Implantation von Sonden im Herz, beispielsweise zur Bestimmung eines EKG-Signals mit einem Herzstimulator, ist mit Risiken für den Patienten behaftet und aufwändig. Gleichwohl bilden Patienten ohne Indikation zur Elektrotherapie einen signifikanten Anteil von Patienten mit Herzinsuffizienz.

Wünschenswert ist ein elektromedizinisches Implantat zum Überwachen einer thorakalen Eigenschaft eines Lebewesens, das auch eine langfristige, vergleichsweise einfache Überwachung ermöglicht, ohne dass eine kardiale Invasivität geboten ist.

WO 2009/035596 A1 und WO 2004/047638 A1 offenbaren medizinische Implantate, die eine thorakale Eigenschaft durch Messung einer Impedanz zwischen am oder im Herzen angeordneter Elektroden und dem Gehäuse des Implantates bestimmen.

Ein dazu eingangs genanntes elektromedizinisches Implantat ist beispielsweise aus US 2009/0062667 A1 bekannt, bei welchem letztlich zur Überwachung eines arteriellen Blutdrucks eine Pulswellenlaufzeit aus Pulsspitzenankunftszeiten zweier Signale bestimmt wird. Mittels subkutan implantierter Elektroden wird ein erstes Signal in Form eines Elektrokardiogramms ermittelt, welches indikativ für eine elektrische Aktivität eines Patientenherzens ist, nämlich den Blutauswurf aus dem Herzen. Zusätzlich wird ein pektoral implantierter Sensor in Form eines Photoplethysmografiesensors genutzt, um ein entsprechendes Signal zu ermitteln, welches indikativ für eine mechanische Aktivität eines Patientenherzens, nämlich die pektorale Ankunft eines Blutpulses, ist. Dieses Konzept kann als ein implantierbarer Monitor ohne Elektrotherapievorrichtung realisiert werden.

US 5,331,966 offenbart ein elektromedizinisches Implantat mit einer Detektoranordnung, die zum Erfassen elektrischer kardialer Signale eine Matrix von relativ nah benachbarten subkutanen Elektroden aufweist, wobei ein ausgewähltes Elektrodenpaar zur Aufnahme eines sogenannten Fernfeld-Elektrokardiogramms vorgesehen ist. Die Matrix von Elektroden ist auf oder in der Nähe des Implantats außerhalb eines Patientenherzens angebracht, wobei die Elektroden in kreisförmiger Form und in einer Dreieckanordnung auf der Oberfläche des Implantats angeordnet sind, um ein vektorielles subkutanes Femfeld-Elektrokardiogramm aufzunehmen.

US 2008/0234591A1 und US 2008/0183225A1 offenbaren ein elektromedizinisches Implantat mit einer Umhüllung, welche das Implantat umgibt und wiederum drei auf einer Schmalseite des Implantats angeordnete Elektroden in ovaler Form aufweist, die zur Aufnahme eines extrakardialen Fernfeld-Kardiogramms vorgesehen sind.

US 2004/0225329A1 offenbart ein elektromedizinisches Implantat mit einer Anzahl von subkutanen Elektroden, die als Teil einer Matrix auf einer Seitenfläche des Implantats angeordnet sind und die zur Aufnahme eines subkutanen Elektrokardiogramms mit auf dem Thorax des Patienten angebrachten Oberflächenelektroden zusammenwirken.

Während sich obige Ansätze von subkutanen elektromedizinischen Implantaten als grundsätzlich sinnvoll zum Überwachen einer thorakalen Eigenschaft eines Lebewesens erweisen, so sind diese verbesserungswürdig.

Der Erfindung liegt die Aufgabe zugrunde, ein elektromedizinisches Implantat, insbesondere ein reines Monitoringimplantat, zum Überwachen einer thorakalen Eigenschaft eines Lebewesens anzugeben, mit dem auf vergleichsweise einfache und dennoch verlässliche Weise eine thorakale Eigenschaft des Lebewesens ermittelt werden kann. Das Implantat soll möglichst kompakt und einfach realisierbar sein.

Aufgabe der Erfindung ist es auch, ein Überwachungssystem unter Nutzung des elektromedizinischen Implantats anzugeben, mit dem die thorakale Eigenschaft, insbesondere aus der thorakalen Eigenschaft ableitbare weitere Patientenwerte, dauerhaft bzw. über einen längeren Zeitraum überwachbar und abrufbar sind. Insbesondere soll ein Überwachungssystem in der Lage sein, durch das elektromedizinische Implantat, angezeigte Patientenwerte für eine Visualisierung verfügbar zu machen. Dadurch kann ein Patientenzustand besonders leicht abgeleitet werden.

Betreffend das elektromedizinische Implantat wird die Aufgabe durch die Erfindung mittels einem elektromedizinischen Implantats der eingangs genannten Art gelöst, bei dem erfindungsgemäß vorgesehen ist:
- eine Detektoranordnung (10) mit einer Impedanzmesseinheit (12) und einer Elektrodenanordnung (11), die eingerichtet sind zum Erfassen eines der thorakalen Eigenschaft zugeordneten Messsignals in Form eines Impedanzsignals;
- eine an der Detektoranordnung (10) angeschlossene Überwachungsanordnung (20), die eingerichtet ist, einen Parameter aus dem Messsignal zu erzeugen, der indikativ für die thorakale Eigenschaft ist, und
- eine an der Überwachungsanordnung (20) angeschlossene Auswerteeinheit (30), die eingerichtet ist, ein Auswerteergebnis zur thorakalen Eigenschaft aus dem Parameter zu bestimmen; wobei
- die Elektrodenanordnung (11) wenigstens eine auf dem Gehäuse (50) angeordnete Anzahl von gegeneinander isolierten, über die Impedanzmesseinheit (12) wirkverbundene und separat ansteuerbare Elektroden (11.1, 11.2, 11.3) aufweist, wobei ein Elektrodenkörper eine Streifenform hat.

Unter einem Impedanzsignal ist vorliegend insbesondere ein Signal einer Spannungsabtastung bei bekannter Stromaufprägung oder ein Signal einer Stromabtastung bei bekannter Spannungsaufprägung oder ein ähnliches Signal zu verstehen, mit dem auf die Impedanz zwischen zwei Polen der Elektrodenanordnung, nämlich das Impedanzsignal, geschlossen werden kann. Insbesondere betrifft dies die Impedanz bei einer bestimmten Frequenz eines elektrischen Stroms bzw. einer elektrischen Spannung. Die Impedanz kann auch aus einem pulsförmigen Wechselstrom bzw. einer pulsförmigen Wechselspannung ermittelt sein. Vorteilhaft weist das elektromedizinische Implantat dazu eine zweckmäßig ausgelegte Impedanzmesseinheit auf. Vorzugsweise weist die Impedanzmesseinheit ein Erregermodul zum Abgeben eines elektrischen Stroms über die Elektrodenanordnung und ein Tastmodul zum Aufnehmen einer Spannung über die Elektrodenanordnung auf. Es kann umgekehrt das Erregermodul auch zum Abgeben einer Spannung über die Elektrodenanordnung und das Abtastmodul zum Aufnehmen eines Stroms über die Elektrodenanordnung genutzt werden. Durch Abgabe eines Stroms oder einer Spannung wird diese dem umliegenden Gewebe aufgeprägt. Umgekehrt kann eine vom umliegenden Gewebe getragene Spannung oder Strom abgetastet werden. Ein Parameter der thorakalen Eigenschaft oder Auswerteergebnisse einer Auswertung des Parameters können durch das elektromedizinische Implantat vorteilhaft einem Überwachungssystem (200) zur Verfügung gestellt werden. Dazu besitzt das elektromedizinische Implantat vorzugsweise ein Kommunikationsmodul zur drahtlosen Übermittlung des Auswerteergebnisses vom Implantat an das Überwachungssystem.

Insgesamt geht die Erfindung von der Überlegung aus, dass sich eine Impedanzmessung besonders vorteilhaft eignet, um Leitfähigkeitsänderungen in dem Thorax eines Lebewesens festzustellen. Weiter geht die Erfindung von der Überlegung aus, dass sich Leitfähigkeitsänderungen in dem Thorax eines Lebewesens eignen, Änderungen beim Flüssigkeitsstatus des Lebewesens in dem Thorax frühzeitig zu erkennen. Das elektromedizinische Implantat eignet sich damit besonders vorteilhaft zum Überwachen eines Flüssigkeitsstatus in dem Thorax eines Lebewesens. In dieser Form können besonders frühzeitig Flüssigkeitsansammlungen, Ödeme oder dergleichen in dem Thorax, insbesondere in einer Lunge, und deren Änderung wirksam überwacht werden. Die Erfindung beruht auf der Erkenntnis, dass sich eine Impedanzmessung erfindungsgemäß am besten mit einer Elektrodenanordnung realisieren lässt, die - vorzugsweise regelmäßig oder unregelmäßig in einer definierten Struktur - eine Anzahl von gegeneinander isolierten Elektroden aufweist. Da die Elektroden über die Impedanzmesseinheit wirkverbunden sind, lassen sich diese, vorzugsweise als Teil einer regelmäßigen Struktur, besonders flexibel und bedarfsgerecht einzeln, paarweise oder in Gruppen aktivieren und zur Impedanzmessung heranziehen. Leitfähigkeitsänderungen im Thorax des Lebewesens lassen sich somit über einzelne oder mehrere redundante oder sich einander ergänzende Kombinationen von Impedanzmessungen mit unterschiedlichen Elektroden einer regelmäßigen Struktur überwachen. Grundsätzlich lassen sich so sehr differenzierte Aussagen über Leitfähigkeitsänderungen in verschiedenen Bereichen des Thorax treffen. Zudem beruht die Erfindung auf der Erkenntnis, dass ein Elektrodenkörper, d.h. der für einen Gewebe- oder Blutkontakt vorgesehene Oberflächenabschnitt einer Elektrode, in einer länglichen Form sich besonders eignet, ein zuverlässiges Impedanzsignal zu ermitteln. Es hat sich gezeigt, dass die längliche Form vor allem eine von der Lage des elektromedizinischen Implantats weitgehend unabhängige Ermittlung des Impedanzsignals ermöglicht.

Im Rahmen einer besonders bevorzugten Weiterbildung kann das elektromedizinische Implantat ein Verankerungsmittel aufweisen. Ein Verankerungsmittel ist vorteilhaft in Form einer Einnähöse, einer Schraube, einer Ankerstruktur oder als ein sonstiges Mittel vorgesehen, um das elektromedizinische Implantat nach subkutaner Implantation in seiner Lage zu fixieren. So kann in diesem implantierten Zustand nicht nur die Lage des elektromedizinischen Implantats stabilisiert werden und eine Migration desselben verhindert werden, sondern auch Bewegungsartefakte in Messsignalen vermieden werden. Die Verankerungsmittel sind vorteilhaft reversibel ausgeführt, d.h. so, dass das Verankerungsmittel gelöst werden kann, möglichst ohne Verletzungen am Lebewesen zu hinterlassen. Dadurch kann das Implantat bei Bedarf wieder aus dem Körper des Lebewesens entfernt werden.

Insbesondere bietet das elektromedizinische Implantat die Möglichkeit, einen thorakalen Flüssigkeitsstatus eines Lebewesens, insbesondere eine Flüssigkeitsansammlung im Thorax des Lebewesens, vorzugsweise in der Lunge, zu ermitteln. Das elektromedizinische Implantat bietet auch die Möglichkeit, ein dem kardialen Blutstrom zugeordnetes Messsignal und ein dem epithorakalen, peripheren Blutstrom zugeordnetes Messsignal beim Patienten besonders einfach und zuverlässig zu erfassen.

Eine Impedanzmessung gemäß dem vorgenannten allgemeinen Konzept zur Bestimmung einer Leitfähigkeit im Thorax eines Lebewesens kann in einer speziellen Variante mit dem elektromedizinischen Implantat der Erfindung ausgeführt werden. Als besonders vorteilhaft hat sich das Implantat zum Überwachen einer thorakalen Eigenschaft in Form eines kardialen Blutstroms und/oder eines epithorakalen, peripheren Blutstroms erwiesen. Dazu kann die Detektoranordnung zum Erfassen eines dem kardialen Blutstrom zugeordneten ersten Messsignals und eines dem epithorakalen, peripheren Blutstrom zugeordneten zweiten Messsignals eingerichtet sein. Darüber hinaus ist die an der Detektoranordnung angeschlossene Überwachungsanordnung eingerichtet, einen ersten Parameter aus dem ersten Messsignal zu erzeugen, der indikativ für einen Zeitpunkt eines Blutauswurfs aus dem Herzen ist und/oder eingerichtet, einen zweiten Parameter aus dem zweiten Messsignal zu erzeugen, der indikativ für einen Zeitpunkt eines Blutpulses in dem thorakalen Gewebe ist, der dem Blutauswurf zugeordnet ist. Die an der Überwachungsanordnung angeschlossene Auswerteeinheit ist dazu eingerichtet, eine Pulswellenlaufzeit aus dem ersten Parameter und dem zweiten Parameter zu erzeugen. Bei dieser Variante sind die Impedanzmesseinheit und die Elektrodenanordnung eingerichtet, das erste und/oder zweite Messsignal in Form eines Impedanzsignals zu detektieren.

Der erste und der zweite Parameter sind jeweils für thorakale Impedanz indikative Parameter. Unter einem Parameter ist vorliegend jedes quantifizierbare Merkmal eines Messsignals zu verstehen. Vorliegend ist ein Parameter insbesondere dann indikativ für einen Zeitpunkt, wenn er ein ausreichend zeitlich einschränkbares Merkmal des Messsignals definiert. Grundsätzlich kann der Parameter aus dem Messsignal selbst oder auch aus einem aus dem Messsignal, z. B. durch Filterung oder mathematische Ableitung oder dergleichen gewonnenen Signal erzeugt werden. Ein Messsignal liegt insbesondere dann in Form eines Impedanzsignals vor, wenn es sich aus einem aufgrund einer Impedanzmessung gewonnenen Signals unmittelbar oder mittelbar ergibt.

Zur Lösung der Aufgabe wird erfindungsgemäß ein Überwachungssystem vorgeschlagen, aufweisend das elektromedizinische Implantat mit dem vorgenannten Kommunikationsmodul, wobei das Überwachungssystem weiter aufweist:
- eine Empfangseinheit zum Empfangen des Auswertesignals;
- eine Speichereinheit für das Auswerteergebnis.

Das Auswerteergebnis wird über die Speichereinheit - das beim Implantat oder bei der Empfangseinheit vorgesehen sein kann - für eine Weiterverwertung verfügbar gemacht. So kann das Auswerteergebnis gespeichert und vom Arzt bei einer Nachsorgeuntersuchung mit einem geeigneten Abfragegerät interrogiert werden. Eine Interrogation kann aber auch vom Patienten selbst oder von einem autorisierten Servicecenter vorgenommen werden.

Das Konzept der Erfindung ermöglicht vorteilhaft, die durch das Implantat überwachte thorakale Eigenschaft und das daraus bestimmbare Auswerteergebnis für eine weitere Analyse verfügbar zu machen. Damit können weitere Patientenwerte generiert werden, die auf einen Patientenzustand hinweisen. Dazu können geeignete weitere Verarbeitungseinheiten oder Abfrageeinheiten im Rahmen des Überwachungssystems vorgesehen werden, die vorzugsweise drahtlos mit dem Überwachungssystem und/oder dem Implantat, insbesondere mit der Empfangseinheit, kommunizieren können und/oder auf die Speichereinheit zugreifen können.

Eine telemetrische Anbindung des elektromedizinischen Implantats an das Überwachungssystem über das Kommunikationsmodul ermöglicht eine automatische Fernüberwachung des Lebewesens mittels des elektromedizinischen Implantats. Der Patient/das Lebewesen lässt sich somit durch den Arzt oder ein Servicecenter fernüberwachen, es können Alarmzustände aufgrund der übermittelten Auswerteergebnisse generiert werden, die im Servicecenter und/oder direkt beim Arzt angezeigt werden. So kann insbesondere ein Arzt über kritische Patientenzustände eines Lebewesens/Patienten über das Servicecenter benachrichtigt werden, beispielsweise direkt oder mittels weiterer Kommunikationsmittel, wie einer Internetplattform, einer SMS, einer E-Mail, einem Fax oder Ähnlichem.

Dabei hat sich insbesondere die Überwachung eines Lungenzustands, insbesondere eines Lungenwassergehalts als zweckmäßig erwiesen. Ganz allgemein kann die Erkennung schwerwiegender Änderungen einer thorakalen Eigenschaft, primär einer Eigenschaft der Lungen, insbesondere zunehmende Flüssigkeitsansammlungen, dazu dienen, sich anbahnende kardiale Dekompensationen zu erkennen. Ziel kann auch die frühzeitige Erkennung von sich bildenden Ödemen, insbesondere Lungenödemen, sein. Letzteres hat häufig eine kardiale Ursache, z.B. als Folge einer Dekompensation, kann aber auch andere Ursachen haben. Grundsätzlich hat sich eine Überwachung eines thorakalen der Flüssigkeitsstatus eines Patienten als zweckmäßig erwiesen. Insbesondere betrifft dies Patienten mit Niereninsuffizienz, transplantierter Niere oder Patienten, die unter Diurese stehen. Anbahnende kardiale und andere schwerwiegende Gesundheitsverschlechterungen des Patienten können dadurch frühzeitig erkannt werden.

Als zweckmäßig hat sich auch die Überwachung eines kardialen Blutstroms und eines epithorakalen, peripheren Blutstroms eines Lebewesens erwiesen, um eine sogenannte Pulswellenlaufzeit zu bestimmen. Unter einer Pulswellenlaufzeit ist die Zeitdifferenz zwischen einem Zeitpunkt eines Blutauswurfs aus dem Herzen eines Patienten bis zum Zeitpunkt eines Blutpulses im thorakalen Gewebe zu verstehen, wobei der Blutpuls im thorakalen Gewebe zeitlich verzögert erfolgt und durch den Blutauswurf verursacht ist.

Eine Verringerung der Pulswellenlaufzeit ist beispielsweise ein Indikator für eine verringerte Gefäßelastizität. Damit verbunden sind eine geringe Durchblutung der Koronargefäβe und ein erhöhtes Infarktrisiko. Eine geringe Gefäßelastizität fördert die Verschlechterung des Gesundheitszustandes bei chronischer Herzinsuffizienz. Die Gefäßelastizität beeinflusst auch die Therapiemöglichkeiten bei Hypertonie. Kenntnisse über die Gefäßelastizität und deren Veränderung können die Art und Dosierung von Medikamenten optimieren.

Auch der arterielle Blutdruck bzw. Änderung des Blutdrucks können aus der Pulswellenlaufzeit geschätzt werden, soweit diese über einen ausreichenden Zeitraum bekannt ist. Der Blutdruck ist eine wichtige systematische Größe. Veränderungen des mittleren Blutdrucks, Änderungen des belastungsabhängigen Blutdruckanstiegs oder Blutdruckschwankungen können wichtige Hinweise auf die Entwicklung einer chronischen Krankheit, z.B. der arteriellen Hypertonie oder der Herzinsuffizienz geben.

Das Konzept der Erfindung führt zu einem Verfahren zum Überwachen einer thorakalen Eigenschaft, bei dem
- ein elektromedizinisches Implantat epithorakal implantiert wird und weiter aufweisend die mittels des Implantats durchführbaren Schritte:
- Erfassen eines der thorakalen Eigenschaft zugeordneten Messsignals in Form eines Impedanzsignals, Erzeugen eines Parameters aus dem Messsignal, der indikativ für die thorakale Eigenschaft ist,
- Bestimmen eines Auswerteergebnisses zur thorakalen Eigenschaft, wobei das Messsignal in Form eines Impedanzsignals mit einer Elektrodenanordnung erfasst wird, die wenigstens eine regelmäßige Struktur einer Anzahl von gegeneinander isolierten und über eine Impedanzmesseinheit wirkverbundene Elektroden aufweist, wobei ein Elektrodenkörper eine längliche Form hat.

Vorteilhaft kann im Überwachungssystem eine Triggereinheit zur zeitlich getriggerten Aktivierung des Kommunikationsmoduls, beispielsweise automatisch, ereignisgesteuert oder patienten- oder arztgesteuert, vorgesehen sein. Dadurch lässt sich eine ständige oder bedarfsmäßige Verfügbarkeit der thorakalen Eigenschaft für eine Weiterverarbeitung zu Patientenwerten oder Analyse eines Patientenzustandes sicherstellen.

Vorteilhaft ist im Überwachungssystem eine Verarbeitungseinheit zur Verarbeitung bzw. Umwandlung des Auswerteergebnisses zur thorakalen Eigenschaft in eine für einen Patientenzustand indizierende und/oder Patientenwerte visualisierende Datenform vorgesehen. Im Rahmen einer indizierenden Datenform ermöglicht dies die Darstellung eines beispielsweise für einen kritischen Zustand eines Patienten indikativen Parameters. Eine visualisierende Datenform eignet sich beispielsweise, um einen kritischen Parameter eines Patientenwertes besonders übersichtlich, z.B. in einem Histogramm oder in einer farblichen Darstellung zu visualisieren.

Das Überwachungssystem weist darüber hinaus vorteilhaft eine Abfrageeinheit zum Abfragen wenigstens des Auswerteergebnisses zur thorakalen Eigenschaft von dem elektromedizinischen Implantat und/oder der Speichereinheit auf. Die Abfrageeinheit kann Teil eines Servicecenters oder ein Patientengerät oder ein Arztgerät sein. Alle Beteiligten können mit dem Überwachungssystem und/oder dem Implantat selbst kommunizieren. Die Empfangseinheit kann auch direkt einem Arzt oder einem Patienten oder einem Servicecenter zugeordnet sein.

Weitere vorteilhafte Weiterbildungen des Implantats gemäß der Erfindung sind den Unteransprüchen zu entnehmen und geben im Einzelnen vorteilhafte Möglichkeiten an, das oben erläuterte Konzept im Rahmen der Aufgabenstellung sowie hinsichtlich weiterer Vorteile zu realisieren.

Das Kommunikationsmodul zur drahtlosen Übermittlung des Auswerteergebnisses kann unterschiedlich realisiert sein. Als besonders vorteilhaft hat sich eine Realisierung auf induktiver Basis (beispielsweise mit Spulentelemetrie) auf elektromagnetischer Basis (beispielsweise mittels elektromagnetischer Wellen im Radiofrequenzbereich) oder auf akustischer Basis (beispielsweise mittels Ultraschall) erwiesen.

Vorteilhaft weist die Elektrodenstruktur zur Bildung einer Anzahl von Polen eine Anzahl von Elektroden auf, die als über die Impedanzmesseinheit paarweise zusammenwirkende Elektroden betreibbar sind. Dadurch lassen sich Impedanzmessungen unterschiedlicher Messkonfiguration und mit gegebenenfalls unterschiedlichem Informationsgehalt gleichzeitig alternierend oder sequentiell realisieren. Bevorzugt kann ein einziges zusammenwirkendes Paar von Elektroden vorgesehen sein. Diese Variante eignet sich vor allem für eine sogenannte Zweipolmessung, bei welcher über die gleichen Elektroden eine Spannungsabtastung als auch eine Stromaufprägung erfolgt. Vorteilhaft kann die Elektrodenstruktur zur Bildung dreier Pole wenigstens einen Elektrodenkörper aufweisen, der über die Impedanzmesseinheit paarweise mit einer ersten und einer weiteren Elektrode zusammenwirkt. Diese Anordnung eignet sich vorteilhaft für eine sogenannte Dreipolmessung, bei welcher eine Spannungsabtastung über eine der Stromaufprägeelektroden und mindestens eine andere Elektrode erfolgt. Des Weiteren hat sich eine Elektrodenstruktur als vorteilhaft erwiesen, bei welcher zwei erste Pole über ein erstes Paar von Elektroden und zwei weitere Pole über ein zweites Paar von Elektroden realisiert ist und die über die Impedanzmesseinheit paarweise zusammenwirken. Eine solche Anordnung eignet sich besonders für eine sogenannte Vierpolmessung, bei welcher eine Spannungsabtastung über mindestens zwei nicht an einer Stromaufprägung beteiligten Elektroden erfolgt.

Vorteilhaft ist ein Abstand der Elektroden eines ersten Elektrodenpaares größer als ein Abstand der Elektroden eines zweiten Elektrodenpaares. Aufgrund eines damit von einer Impedanzmessung beeinflussten Gewebeumfangs, kann über das zweite Paar der Elektroden die Impedanz und damit die Leitfähigkeit eines Gewebes in der Nähe des Implantats bestimmt werden, während mit dem ersten Paar der Elektroden eher die Impedanz bzw. die Leitfähigkeit eines Gewebes bestimmt werden kann, das, relativ zum Implantat gesehen, tieferer Regionen des Thorax betrifft.

Mit ähnlichem Vorteil versehen kann im Rahmen einer Weiterbildung die Elektrodenanordnung wenigstens eine nahe des Implantats angeordnete Elektrode umfassen. Die nahe des Implantats angeordnete Elektrode ist insbesondere in Form der Elektrodenstruktur gebildet. Zusätzlich oder alternativ kann die nahe des Implantats angeordnete Elektrode in Form des Gehäuses des Implantats gebildet sein. Eine in unmittelbarer Nähe des Implantats, d.h. auf oder mit dem Gehäuse gebildete Elektrode eignet sich in besonders vorteilhafter Weise für eine Impedanzmessung des das Implantat unmittelbar umgebenden Körpergewebes, z.B. zur Überwachung eines epithorakalen, peripheren Blutstroms.

Vorteilhaft ist auch eine Elektrodenanordnung, welche eine entfernt des Implantats angeordnete weitere Elektrode als Teil einer Elektrodenstruktur umfasst. Mittels einer entfernt des Implantats angeordneten Elektrode lassen sich Impedanzmessungen in tieferen Regionen des Körpergewebes durchführen. Damit kann beispielsweise die Impedanz zur Ermittlung eines Flüssigkeitsstatus aus tieferen Regionen bestimmt werden. Ein solcher Flüssigkeitsstatus aus tieferen Regionen kann zur Bestimmung einer weiteren thorakalen Eigenschaft, z.B. eines Flüssigkeitsstatus der Lunge oder eines kardialen Blutstroms herangezogen werden. Auch kann durch Differenzbildung eines Impedanzsignals für tiefere Regionen des Gewebes und eines Impedanzsignals für naheliegende Regionen des Gewebes ein Signalkorrektor erreicht werden. Beispielsweise kann die Impedanz zur Bestimmung des Lungenwassers aus tieferen Regionen mit der Impedanz in der Umgebung des Implantats korrigiert werden, um Schwankungen der Blutleitfähigkeit, z.B. durch Hämatokritschwankungen, zu kompensieren. Umgekehrt kann die Differenz von Impedanzsignalen betreffend einen kardialen Blutstrom einerseits und eines epithorakalen, peripheren Blutstroms andererseits dazu dienen, einen Parameter zu erzeugen, der indikativ für einen Zeitpunkt eines Blutauswurfs aus dem Herzen ist und einen zweiten Parameter zu erzeugen, der indikativ für einen Zeitpunkt eines Blutpulses in dem thorakalen Gewebe ist.

Vorteilhaft ist eine erste Elektrodenstruktur der Elektrodenanordnung auf dem Gehäuse gebildet und/oder eine zweite Elektrodenstruktur der Elektrodenanordnung auf einem am Gehäuse angeschlossenen Elektrodenkabel gebildet. Die erste Elektrodenstruktur eignet sich bevorzugt zur Bildung einer nahe zum Implantat angeordneten Elektrode. Die zweite Elektrodenstruktur eignet sich bevorzugt zur Bildung einer entfernt des Implantats angeordneten Elektrode. Eine implantatnahe Elektrode hat einen geringeren Abstand zum Implantat als eine implantatferne Elektrode.

Grundsätzlich lässt sich ein Elektrodenkörper länglicher Form auf unterschiedliche Weise im Rahmen des Konzepts der Erfindung bilden. Als besonders vorteilhaft hat sich eine

Elektrodenstruktur erwiesen, die einen Elektrodenkörper in Form einer Streifenelektrode aufweist, d.h. einer Elektrode mit länglicher Erstreckung und einem Anfang und einem Ende. Ein Elektrodenkörper kann auch als Ringelektrode ausgeführt sein, die ein Gehäuse des Implantats umgibt, z.B. als Ring ineinander übergehend oder spiralförmig. Es eignet sich auch ein Elektrodenkörper in Form einer Interdigitalelektrode, wie sie beispielsweise als Fingerelektrode oder sonstige ineinandergreifende Elektrodenstruktur bekannt ist. Die Elektroden können auf einer Seitenfläche, auf einer Rückseite, einer Vorderseite des Gehäuses oder auf allen Seiten des Gehäuses - selbiges umgebend - angebracht sein.

Als besonders geeignet hat sich eine Elektrodenstruktur mit einer Anzahl von äquidistanten Elektrodenkörpern erwiesen. Eine solche Elektrodenstruktur weist vorteilhaft wenigstens drei äquidistante Elektrodenkörper auf. Drei äquidistante Elektrodenkörper eignen sich bereits vorteilhaft zur Umsetzung einer Dreipolmessung. Vier äquidistante Elektrodenkörper können vorteilhaft für eine Vierpolmessung benutzt werden.

Für eine von der Lage des Implantats unabhängige Impedanzmessung eignet sich insbesondere eine Anzahl von symmetrisch zu dem Gehäuse des Implantats angeordneten Elektroden. Insbesondere können die Elektroden mehrseitig oder allseitig oder ringförmig um ein Gehäuse des Implantats angeordnet sein.

Im Rahmen einer besonders bevorzugten Weiterbildung kann eine erste Elektrode und/oder ein erstes Elektrodenpaar der Elektrodenanordnung separat ansteuerbar sein. Mit anderen Worten, ist eine erste Elektrode und/oder ein erstes Elektrodenpaar der Elektrodenanordnung mit einem von einer weiteren Elektrode und/oder weiteren Elektrodenpaar unterschiedlichen Parametern steuerbar. Der Parameter kann beispielsweise eine Frequenz und/oder eine Amplitude und/oder eine Phase für die Impedanzmessung betreffen.

Die Messung der Impedanz (Z) bedeutet die Messung eines komplexen Widerstandes, der einen Realteil und einen Imaginärteil aufweist (Z = R + jX), was auch mittels Betrag (|Z|; sozusagen "Amplitude") und Phase (ϕ) ausgedrückt werden kann. Die Impedanzmesseinheit kann so ausgebildet sein, dass sie beispielsweise einen Strom mit einer definierten Erregerfrequenz (f) aufprägen kann und die Spannung in Betrag und Phasenlage bezüglich des aufgeprägten Stroms messen kann.

Beispielsweise kann ein erstes Messsignal in Form eines ersten Impedanzsignals auf Basis einer ersten Erregerfrequenz gebildet sein und ein zweites Messsignal in Form eines zweiten Impedanzsignals auf Basis einer zweiten Erregerfrequenz gebildet sein. Damit können zusätzlich dielektrischen Eigenschaften des Gewebes bestimmt werden. Aus den dielektrischen Eigenschaften des Gewebes lassen sich zusätzlich Informationen gewinnen, die ebenfalls zur Erhöhung der Sensitivität bzw. Spezifität der Impedanzmessung herangezogen werden können. Darüber hinaus lassen sich aus dielektrischen Gewebeeigenschaften vorteilhaft auch biochemische Informationen gewinnen, beispielsweise über Veränderungen des Glukosespiegels im Blut oder Änderung des Hämatokrits bei Diurese.

Eine Frequenzvariation kann beispielsweise durch einen sinusförmigen Strom mit variabler Frequenz oder durch Strompulse variabler Pulsbreite erfolgen. Eine Messfrequenz bzw. die Variation der Messfrequenzen kann beispielsweise im Bereich von 200 Hz bis 10 GHz liegen.

Es hat sich als vorteilhaft erwiesen, dass eine zweite Erregerfrequenz für das zweite Messsignal niedriger ist als die erste Erregerfrequenz für das erste Messsignal. Es hat sich überraschend gezeigt, dass bei einer vergleichsweise hohen Erregerfrequenz ein zeitlicher Impedanzverlauf messbar ist, bei dem insbesondere die Volumenänderung des Herzens gut erfassbar wird. Nach geeigneter Frequenz-Hochpass-Filterung kann aus einem solchen Impedanzverlauf mit höherer Erregerfrequenz besonders vorteilhaft der Zeitpunkt eines Blutauswurfs aus dem Herzen bestimmt werden. Vorliegend kann dazu insbesondere der Zeitpunkt eines Beginns einer Ventrikelkontraktion bestimmt werden.

Darüber hinaus hat sich gezeigt, dass bei vergleichsweise niedriger Erregerfrequenz ein zeitlicher Impedanzverlauf messbar ist, mit dem die Durchblutung des das Implantat umgebenden Gewebes erfassbar ist, d.h. insbesondere ein zweites Messsignal erzeugbar ist, das indikativ einen Zeitpunkt eines Blutpulses in dem thorakalen Gewebe ist. Nach geeigneter Frequenz-Tiefpass-Filterung kann aus einem solchen Impedanzverlauf der Zeitpunkt eines Eintreffens einer Blutdruckwelle in einem thorakalen Gefäß bestimmt werden.

Eine das erfindungsgemäße Konzept in besonders vorteilhafter Weise weiterbildende Kombination sieht entsprechend vor, dass ein zweites Messsignal in Form eines zweiten Impedanzsignals auf Basis einer zweiten Erregerfrequenz und mit einer implantatnahen Elektrode der Elektrodenanordnung gebildet ist und ein erstes Messsignal in Form eines ersten Impedanzsignals auf Basis einer ersten Erregerfrequenz mit einer implantatfernen Elektrode der Elektrodenanordnung gebildet ist, wobei die zweite Erregerfrequenz niedriger als die erste Erregerfrequenz ist.

Eine über der ersten und zweiten Erregerfrequenz liegende Hochfrequenz, wenigstens im MHz- oder GHz-Bereich kann insbesondere zur Messung einer Hintergrundimpedanz genutzt werden.

Mit Vorteil versehen umfasst die Detektoranordnung auch eine EKG-Messeinheit mit einer Elektrodenanordnung, die eingerichtet ist, eine thorakale Eigenschaft in Form eines Elektrokardiogramms (EKG-Signals) zu detektieren, d.h. subkutan zu messen. Wenn drei oder mehr Elektroden vorhanden sind, kann das EKG in mehreren Ableitungen bestimmt werden. Aus dem EKG-Signal können unter anderem die Herzfrequenz, die QRS-Breite verschiedene Arten von Tachyarrhythmien, Synkopen, usw. diagnostiziert werden. Aus der T-Wellen-Morphologie kann z.B. ein T-Wellen-Alternans als Risikomarker bestimmt werden. Aus dem EKG-Signal können auch RR-Intervalle für aufeinanderfolgende Herzzyklen berechnet werden. Aus einer Zeitreihenanalyse von aufeinanderfolgenden RR-Intervallen können die Herzratenvariabilität und Herzratenturbulenz bestimmt werden. Insbesondere können aus dem EKG-Signal auch thorakale Leitfähigkeitsänderungen detektiert werden, z.B. anhand von Änderungen der Signalamplitude.

Auch kann aus dem EKG-Signal in Kombination mit dem Impedanzsignal - nämlich gemäß der oben genannten speziellen Variante der Erfindung unter Berücksichtigung des oben genannten ersten Parameters und zweiten Parameters eines ersten und zweiten Messsignals - die Pulswellengeschwindigkeit bestimmt werden, um daraus diagnostische Aussagen über den Krankheitszustand anhand des Zustands der arteriellen Gefäße zu treffen. Die Pulswellengeschwindigkeit kann dann aus der Zeitdifferenz zwischen der R-Zacke des QRS-Komplexes im EKG-Signal und dem Volumenpuls im Blutgefäß, der sich im Impedanzsignal widerspiegelt, bestimmt werden.

Vorteilhaft weist die Detektoranordnung zum Erfassen einer thorakalen Eigenschaft einen oder mehrere der Detektoren auf, die ausgewählt sind, aus der Gruppe bestehend aus: akustischer Detektor, optischer Detektor, Druck- und/oder Dehnungsdetektor, Beschleunigungsdetektor, Temperaturdetektor, Sauerstoffdetektor, biochemischer Detektor.

Durch solche und andere Detektoren können eine Vielzahl weiterer sinnvoller Informationen für eine thorakale Eigenschaft gewonnen werden.

Insbesondere hat sich ein Beschleunigungsdetektor als vorteilhaft erwiesen. Mit einem in dem Implantatgehäuse integrierten Beschleunigungssensor kann die Aktivität des Patienten und deren Änderung beobachtet werden. Eine Aktivitätsinformation kann mit einem Impedanz- und/oder EKG-Signal kombiniert werden, um beispielsweise Ruhe- und Aktivphasen zu diskriminieren und Auswerteergebnisse entsprechend zu analysieren. Vorteilhaft ist ein Beschleunigungsdetektor als Dreiachssensor ausgeführt, so dass z.B. bei bekannter Lage des Implantats die momentane Körperposition des Patienten bestimmt werden kann. Anhand eines dreidimensionalen Beschleunigungssignals können Stürze des Patienten detektiert werden. Ein Beschleunigungsdetektor kann gleichzeitig auch als akustischer Detektor ausgeführt werden, wenn dessen Bandbreite hinreichend groß ist, um akustische Signale im hörbaren oder superhörbaren Frequenzbereich zu erfassen.

Ein akustischer Detektor hat sich beispielsweise in Form eines Mikrofons bewährt. Mit einem akustischen Detektor können Lungen- und Herzgeräusche erfasst werden. Lungen-, Herz- und Atemgeräusche können gegebenenfalls im Zusammenhang mit der Impedanzmessung gegebenenfalls auch im Zusammenhang mit einem EKG, als weitere Hinweise zur Erkennung von Lungenödemen, Apnoen oder anderer Lungenerkrankungen dienen. Herzgeräusche können wichtige diagnostische Informationen liefern, z.B. ist bekannt, dass der dritte Herzton zur Überwachung von Herzinsuffizienz verwendet werden kann. Insbesondere mit der EKG-Messung können anhand der Herzgeräusche beispielsweise die Klappenschlusszeitpunkte bestimmt werden.

Von den weiteren vorgenannten Detektoren hat sich insbesondere ein Temperaturdetektor zur Messung der Körpertemperatur des Lebewesens als vorteilhaft erwiesen. Ein Sauerstoffdetektor kann sich zur Messung einer Sauerstoffsättigung im umliegenden Gewebe als vorteilhaft erweisen, z.B. in Form eines optischen Detektors, der am oder im Implantat angebracht ist. Biochemische Detektoren können ebenfalls vorteilhaft im Implantat integriert sein. So können biochemische Detektoren, Sensoren für Glukose, Elektrolyte, Hormone oder Biomarker enthalten und zusätzliche wertvolle Informationen über den Gesundheitszustand des Patienten liefern.

Ausführungsbeispiele der Erfindung werden nun nachfolgend anhand der Zeichnung beschrieben. Diese soll die Ausführungsbeispiele nicht notwendigerweise maßstäblich darstellen, vielmehr ist die Zeichnung, wo zur Erläuterung dienlich, in schematisierter und/oder leicht verzerrter Form ausgeführt. Im Hinblick auf Ergänzungen der aus der Zeichnung unmittelbar erkennbaren Lehren wird auf den einschlägigen Stand der Technik verwiesen. Dabei ist zu berücksichtigen, dass vielfältige Modifikationen und Änderungen betreffend die Form und das Detail einer Ausführungsform vorgenommen werden können, ohne von der allgemeinen Idee der Erfindung abzuweichen. Die in der Beschreibung, in der Zeichnung sowie in den Ansprüchen offenbarten Merkmale der Erfindung können sowohl einzeln als auch in beliebiger Kombination für die Weiterbildung der Erfindung wesentlich sein. Zudem fallen in den Rahmen der Erfindung alle Kombinationen aus zumindest zwei der in der Beschreibung, der Zeichnung und/oder den Ansprüchen offenbarten Merkmale. Die allgemeine Idee der Erfindung ist nicht beschränkt auf die exakte Form oder das Detail der im folgenden gezeigten und beschriebenen bevorzugten Ausführungsform oder beschränkt auf einen Gegenstand, der eingeschränkt wäre im Vergleich zu dem in den Ansprüchen beanspruchten Gegenstand. Bei angegebenen Bemessungsbereichen sollen auch innerhalb der genannten Grenzen liegende Werte als Grenzwerte offenbart und beliebig einsetzbar und beanspruchbar sein. Der Einfachheit halber sind nachfolgend für identische oder ähnliche Teile oder Teile mit identischer oder ähnlicher Funktion gleiche Bezugszeichen verwendet.

Weitere Vorteile, Merkmale und Einzelheiten der Erfindung ergeben sich aus der nachfolgenden Beschreibung der bevorzugten Ausführungsbeispiele sowie anhand der Zeichnung; diese zeigt in:
- Fig. 1: eine symbolische Darstellung eines Überwachungssystems gemäß einer bevorzugten Ausführungsform der Erfindung in einem symbolisch dargestellten Thorax eines Lebewesens;
- Fig. 2: das elektromedizinische Implantat beim Überwachungssystem der Fig. 1 in einer besonders bevorzugten Funktionsstellung, nämlich mit Elektrodenpaaren, die Elektroden mit unterschiedlichen Abständen aufweisen, wobei die Elektrodenpaare mit Impedanzmessungen bei unterschiedlicher Frequenz genutzt werden können, so dass sich unterschiedliche Messreichweiten ergeben.

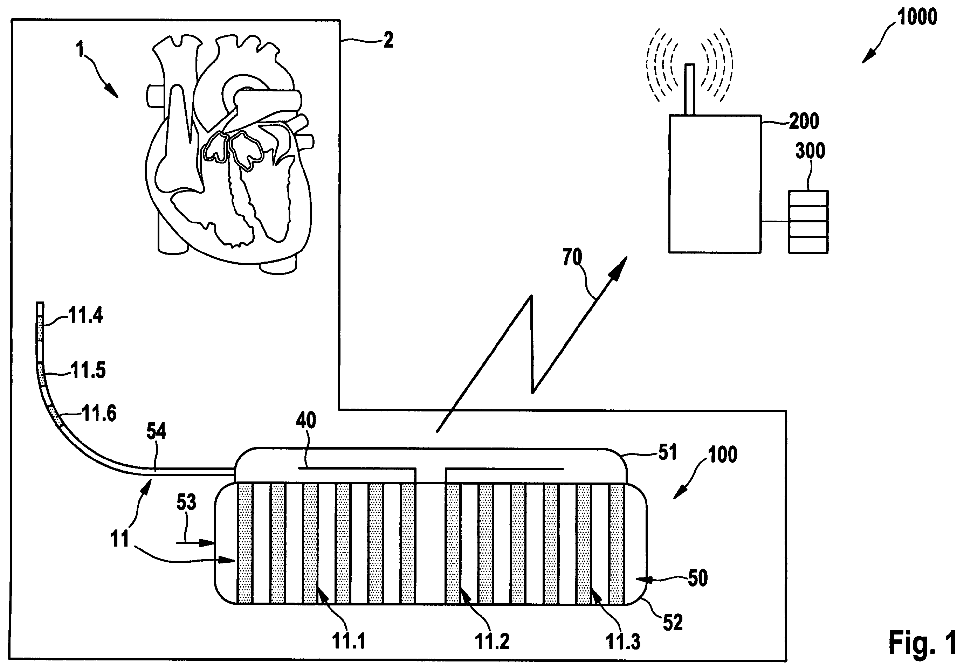

Fig. 1 zeigt eine symbolische Darstellung einer bevorzugten Ausführungsform eines Überwachungssystems 1000 mit einem elektromedizinischen Implantat 100, das zum Überwachen einer thorakalen Eigenschaft eines Lebewesens, nämlich regelmäßig eines Patienten mit oder ohne Indikation auf eine Herzinsuffizienz, eingerichtet ist - das Herz 1 des Patienten und eine Thorax 2 des Patienten sind symbolisch dargestellt. Das Implantat 100 ist vorliegend als reines Monitoringimplantat, d.h. ohne Therapiefunktion ausgelegt. Das Implantat 100 hat ein Gehäuse 50 mit einem Gehäusebehälter 52 und einem sogenannten Header 51 sowie eine am Gehäuse 50 angeschlossene Elektrodenanordnung 11. Das elektromedizinische Implantat 100 kann über ein am Header 51 angeordnetes Kommunikationsmodul 40, nämlich vorliegend ein RF-Antenne, eine drahtlose Telemetrieverbindung 70 zu einer Empfangseinheit 200 des Überwachungssystems 1000 aufbauen. Ein Parameter, der indikativ für die thorakale Eigenschaft ist und/oder ein Auswerteergebnis aus dem Parameter kann so in einer Speichereinheit 300 des Überwachungssystems 1000 verfügbar gemacht werden. Das Auswerteergebnis kann so durch eine nicht näher erläuterte Prozessoreinheit im Überwachungssystem 1000 weiterverarbeitet werden, z.B. zu weiteren Patientenwerten, die indikativ für einen Patientenzustand sind. Solche Patientenwerte können auch vorteilhaft visualisiert werden, beispielsweise im Rahmen von Histogrammen, transienten Signalen oder Mittelwerten, um einem Arzt, Patienten oder Servicecenter auf möglichst intuitive und schnell erkennbare Weise kritische Zustände des Patienten zu verdeutlichen. Auf diese Weise können Alarmzustände einem Arzt, dem Patienten oder dem Servicecenter selbst verfügbar gemacht werden, indem der Arzt, der Patient oder das Servicecenter Zugriff auf den Speicher 300 des Überwachungssystems 1000 hat. Vorliegend wird der Zugriff über ein nicht näher gezeigtes mobiles oder internetbasiertes Netzwerk auf das Überwachungssystem 1000 ermöglicht. Individuelle Patientenwerte bzw. ein individueller Patientenzustand kann für den Arzt oder das Servicecenter oder den Patienten selbst mittels Internetmasken visualisiert werden. Vorliegend werden Patientenwerte in einem Servicecenter automatisch analysiert und bedrohliche Zustände bzw. eine Verschlechterung eines Gesundheitszustands, etc. diagnostiziert und dem Arzt angezeigt. Teilweise kann eine solche Anzeige auch in der Empfangseinheit 200 erfolgen, von der auch eine Anzahl vorhanden sein kann, z.B. als Patientengerät, als Arztgerät oder als sonstiges Betreuergerät.

Das vorliegend gezeigte Überwachungssystem 1000 ermöglicht die Fernüberwachung eines thorakalen Flüssigkeitsstatus bei einem Patienten mittels des Implantats 100. Mittels einer über die Elektrodenanordnung 11 durchgeführten Impedanzmessung werden Leitfähigkeitsänderungen in der Thorax 2 des Patienten detektiert und so Flüssigkeitsansammlungen erkannt, die unter anderem Aufgrund einer sich anbahnenden kardialen Dekompensation auftreten können. Dies betrifft insbesondere Flüssigkeitsansammlungen in einer Lunge des Patienten. Die Impedanzmessung erfolgt durch Aufprägen eines elektrischen Stroms, im Beispielsfall mittels eines pulsförmigen Wechselstroms, mit konstanter Amplitude und Abtasten einer resultierenden Spannung über die Elektrodenanordnung 11 des Implantats 100. Dazu wird mittels des ermittelten Impedanzsignals auch eine Atmungsinformation, nämlich eine Atemamplitude und Frequenz und daraus ein Atemminutenvolumen bestimmt. Kurzfristige Signalschwankungen können ausgewertet werden und z.B. Schlafapnoen detektiert werden oder ein Apnoeindex berechnet werden. Insbesondere dient vorliegend die Detektion einer Atmungsinformation dazu, die Impedanzmessung in dem Thorax 2 des Patienten auf den Atemzyklus zu synchronisieren, um so den Einfluss der Atmung auf die Impedanzmessung zu eliminieren. Außerdem wird über die Elektrodenanordnung 11 eine epithorakale, periphere EKG-Messung als subkutanes EKG durchgerührt, um einen Herzzyklus zu bestimmen. Die Impedanzmessung als solche kann dann auch auf den Herzzyklus synchronisiert werden, so dass herzzyklusabhängige Einflüsse kompensiert oder speziell analysiert werden können.

Die Elektrodenanordnung 11 ist vorliegend zum einen in einer ersten Elektrodenstruktur mit einer Anzahl von gegeneinander isolierten, über eine Impedanzmesseinheit wirkverbundene und separat ansteuerbare Elektroden 11.1, 11.2, 11.3 versehen, die Leitfähigkeitsänderungen über eine Impedanzmessung detektieren können und so dazu dienen, einen Flüssigkeitsstatus des Patienten anzuzeigen. Die implantatnahen Elektroden 11.1, 11.2, 11.3 sind beispielhaft für die Gesamtzahl der Elektroden der ersten Elektrodenstruktur auf dem Gehäuse 50 des Implantats genannt. Ein Elektrodenkörper der Elektroden 11.1, 11.2, 11.3 hat eine längliche Form, z. B. eine Streifenform. Die Elektroden 11.1, 11.2, 11.3 der ersten Elektrodenstruktur sind vorliegend als Ringelektroden ausgebildet und umgeben den Gehäusebehälter 52 des Gehäuses 50 des Implantats 100 ringförmig. Ein solcher ringförmiger Elektrodenkörper hat sich als besonders geeignet erwiesen, um Lageeinflüsse des Implantats 100 auf eine Impedanzmessung weitgehend zu vermeiden. Eine Ringelektrode hat insofern die Eigenschaft, symmetrisch zum Implantat angeordnet zu sein, so dass unabhängig von einer Drehung des Implantats 100 zu seiner Achse die Impedanzmessung zur Bestimmung einer thorakalen Leitfähigkeit erfolgen kann. Außerdem ist das Implantat 100 mit einer reversiblen Verankerung 53 versehen, um dessen Lage zu stabilisieren und eine Migration des Implantats 100 zu vermeiden.

Die weiteren zur Bestimmung eines subkutanen EKG-Signals mittels einer weiteren epithorakalen, peripheren Impedanzmessung vorgesehenen Elektroden 11.4, 11.5, 11.6 sind als Teil einer zweiten Elektrodenstruktur gegeneinander isoliert und über die Impedanzmesseinheit wirkverbunden und an einem am Header 51 angeordneten Kabel 54 platziert. Alle Elektroden 11.1, 11.2, 11.3 der ersten Elektrodenstruktur und alle Elektroden 11.4, 11.5, 11.6 der zweiten Elektrodenstruktur sind bevorzugt aber nicht zwingend jeweils äquidistant zueinander angeordnet. Aufgrund der proximalen Lage der Elektroden 11.1, 11.2, 11.3 der ersten Elektrodenstruktur lässt sich besonders gut eine epithorakale, periphere Impedanz durch das Implantat 100 bestimmen. Für die epithorakale, periphere Impedanzmessung durch die erste Elektrodenstruktur dient ein Gewebeumfang, der weitgehend in unmittelbarer Nähe des Implantats 100 liegt, mit der Reichweite eines aufgeprägten Stroms bzw. abgetasteten Spannungssignals in dem umliegenden Gewebe zum Implantat zusammenhängt. Dagegen dienen die drei über das Kabel 54 abgesetzten Elektroden 11.4, 11.5, 11.6 der zweiten Elektrodenstruktur als implantatferne Elektroden und können ein kardiales, subkutanes EKG ermitteln. Aufgrund der Anzahl von drei Elektroden lässt sich das EKG auch in mehreren Ableitungen bestimmen.

Aus dem EKG-Signal kann zusätzlich zu der Impedanzmessung durch die erste Elektrodenstruktur auf thorakale Leitfähigkeitsänderungen geschlossen werden - neben üblichen Patientenwerten, wie Herzfrequenz, QRS-Breite, verschiedene Arten von Tachyarrhythmien, Synkopen, T-Wellen-Morphologie und R-Intervallen. Vorliegend geschieht dies anhand von Änderungen der EKG-Signalamplitude. In dieser Funktionsstellung dient das elektromedizinische Implantat 100 - basierend auf einer Impedanzmessung, zur thorakalen Leitfähigkeitsbestimmung bei einem Patienten - dazu, um eine Flüssigkeitsansammlung in dem Thorax 2 des Patienten frühzeitig zu erkennen.

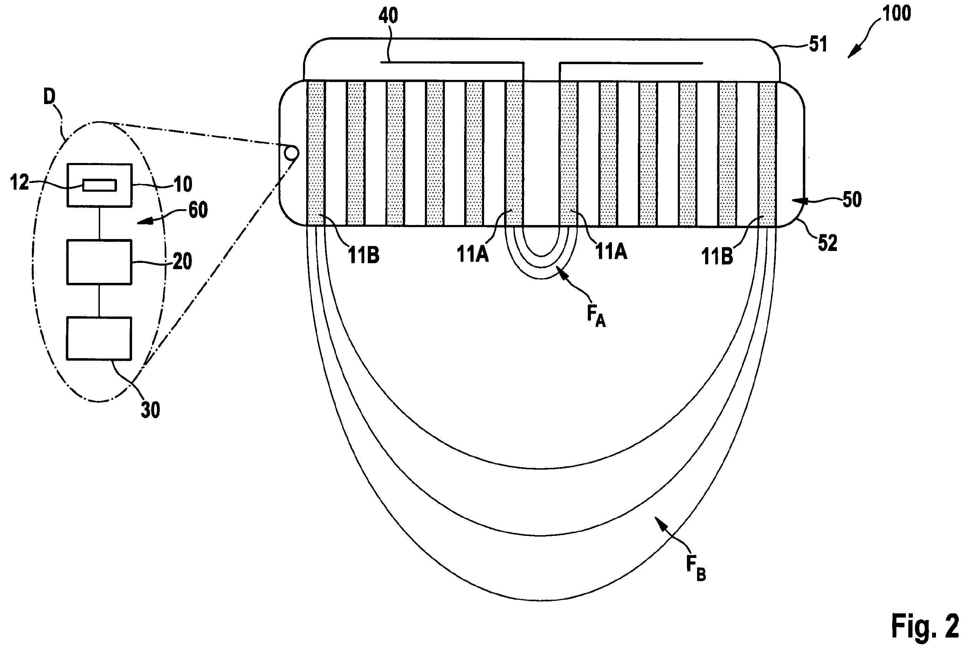

Fig. 2 zeigt dazu eine besonders bevorzugte erste funktionelle Einstellung der ersten Elektrodenstruktur, bei der Elektroden eines ersten Elektrodenpaares 11A und Elektroden eines zweiten Elektrodenpaares 11B über die Impedanzmesseinheit wirkverbunden sind, d.h. jeweils zur Bildung eines Polpaares für eine Zweipolmessung genutzt werden. Über die Elektroden des Elektrodenpaares 11A wird sowohl ein Strom aufgeprägt als auch eine Spannung abgetastet - gleiches gilt für das Elektrodenpaar 11B. Dabei weisen die Elektroden des ersten Elektrodenpaares 11A Vergleich zu den Elektroden des zweiten Elektrodenpaares 11B einen geringeren Abstand auf. Dies führt dazu, dass die beispielhaft eingezeichneten für die Impedanzmessung wirksamen Feldlinien F_{A} eines Spannungsverlaufs des ersten Elektrodenpaares 11A nur eine vergleichsweise geringe Eindringtiefe im Gewebe haben - dies im Vergleich zu analogen Feldlinien F_{B} des zweiten Elektrodenpaares 11_{B}. Bereits dieser Umstand führt dazu, dass mit einer Impedanzmessung über das erste Elektrodenpaar 11A die Impedanz in unmittelbarer Nähe des Implantats bestimmt wird. Mit einer Impedanzmessung über das zweite Elektrodenpaar 11B wird eine Impedanz in tieferen Regionen des Körpergewebes gemessen. Vorliegend wird die Impedanz zur Bestimmung des Lungenwassers aus tieferen Regionen mit der Impedanz in der Umgebung des Implantats korrigiert, um Schwankungen der Blutleitfähigkeit, z.B. durch Hämatokritschwankungen, zu kompensieren.

Eine noch weiter verfeinerte Impedanzmessung sieht vor, dass eine Impedanzmessung über das erste Elektrodenpaar 11 A bei niedrigerer Frequenz erfolgt als eine Impedanzmessung über das Elektrodenpaar 11B. Vorliegend wird die Impedanzmessung über das Elektrodenpaar 11A bei 2 kHz durchgeführt, während die Impedanzmessung über das Elektrodenpaar 11B bei 20 kHz durchgeführt wird. Die Impedanzsignale lassen sich über einen Frequenz-Tiefpass-Filter für das Elektrodenpaar 11A und über einen Frequenz-Hochpass-Filter für das Elektrodenpaar 11B separieren. Es hat sich gezeigt, dass der niederfrequente Impedanzsignalanteil indikativ für einen Zeitpunkt eines Blutpulses in dem das Implantat unmittelbar umgebenden thorakalen Gewebe sein kann.

In einer abgewandelten zweiten Funktionsstellung lässt sich aus dem Impedanzsignalverlauf, wie er mit dem ersten Elektrodenpaar 11A gewonnen wird, der Zeitpunkt eines Eintreffens einer Blutdruckwelle in einem thorakalen Gefäß bestimmen. Darüber hinaus hat sich gezeigt, dass aus dem Impedanzsignalverlauf bei höherer Frequenz der Zeitpunkt eines Blutauswurfs aus dem Herzen bestimmt werden kann, nämlich vorliegend der Zeitpunkt eines Beginns einer Ventrikelkontraktion. Letzterer Umstand ergibt sich aus dem Eindringen der für die Impedanzmessung relevanten Feldlinien F_{B} in tiefere Regionen der Thorax im Vergleich zu den Feldlinien F_{A} der Impedanzmessung über das erste Elektrodenpaar 11 A.

Aus der Differenz des Zeitpunkts t_{gef} eines Eintreffens einer Blutdruckwelle in einem thorakalen Gefäß und des Zeitpunkts t_{sys} eines Blutauswurfs aus dem Herzen lässt sich die sogenannte Pulswellenlaufzeit delta-t (Δt) bestimmen. Letztere ist ein guter Indikator für einen Gefäßzustand oder einen Blutdruck des Patienten. In dieser zuvor beschriebenen zweiten Funktionsstellung mit frequenzabhängiger Impedanzmessung beim elektromedizinischen Implantat lassen sich somit Aussagen über einen kardialen Blutstrom und einen epithorakalen, peripheren Blutstrom treffen, um eine Pulswellenlaufzeit zu bestimmen.

In einer dritten aus dem Aufbau der Fig. 1 ersichtlichen Funktionsstellung des elektromedizinischen Implantats 100 kann ebenfalls auf die Pulswellenlaufzeit geschlossen werden. Dazu wird der Zeitpunkt eines Blutauswurfs aus dem Herzen, nämlich der Beginn der Ventrikelkontraktion aus einer R-Zacke des QRS-Komplexes des EKG-Signals bestimmt - letzteres über die Elektroden 11.4, 11.5, 11.6 der zweiten Elektrodenstruktur am Kabel 54 gemessen. Zusätzlich mit einem aus der Impedanzmessung der Elektroden 11.1, 11.2, 11.3 der ersten Elektrodenstruktur erhältlichen Zeitpunkt eines Eintreffens eines Blutpulses in dem thorakalen Gewebe lässt sich - wiederum als Differenz der Zeitpunkte - eine Pulswellenlaufzeit bestimmen.

Insgesamt lassen sich unterschiedliche, insbesondere die drei vorgenannten Funktionsstellungen des elektromedizinischen Implantats mit Hilfe einer im vergrößerten Detail D dargestellten Mess- und Steuerelektronik 60 des Implantats 100 einstellen. Die Mess- und Steuerelektronik 60 des Implantats 100 weist insbesondere eine die Impedanzmesseinheit 12 umfassende Detektoranordnung 10 auf, die zusammen mit der Elektrodenanordnung 11, nämlich der Elektroden der ersten Elektrodenstruktur und der zweiten Elektrodenstruktur, zum Erfassen eines der thorakalen Eigenschaft zugeordneten Messsignals in Form eines Impedanzsignals und/oder EKG-Signals ausgebildet ist. Die Mess- und Steuerelektronik 60 umfasst weiter eine an der Detektoranordnung 10 angeschlossene Überwachungsanordnung 20, die eingerichtet ist, einen Parameter aus dem Messsignal zu erzeugen, der indikativ für die thorakale Eigenschaft ist. Bei der thorakalen Eigenschaft handelt es sich in der ersten Funktionsstellung des elektromedizinischen Implantats 100 um eine Leitfähigkeit der Thorax 2, dies zur Ermittlung eines Flüssigkeitsstatus des Patienten. Bei der zweiten und dritten Funktionsstellung handelt es sich um einen Parameter eines kardialen oder epithorakalen, peripheren Impedanz-Messsignals, der auf einen Zeitpunkt eines Blutauswurfs aus dem Herzen bzw. einen Zeitpunkt des Eintreffens eines Blutpulses im thorakalen Gewebe hinweist.

An der Überwachungsanordnung 20 ist eine Auswerteeinheit 30 angeschlossen, die eingerichtet ist, ein Auswerteergebnis zur thorakalen Eigenschaft aus dem Parameter zu quantifizieren. Dies kann im Falle der ersten Funktionsstellung ein Auswerteergebnis in Form einer quantifizierten thorakalen Flüssigkeitsmenge sein. Dies kann im Falle der zweiten oder dritten Funktionsstellung des elektromedizinischen Implantats eine Pulswellenlaufzeit Δt sein, die sich aus der Differenz eines Zeitpunkts des Blutauswurfs aus dem Herzen und eines Zeitpunkts des Eintreffens eines Blutpulses im thorakalen Gewebe ergibt.

Zusammenfassend betrifft die Erfindung ein elektromedizinisches Implantat 100 zum Überwachen einer thorakalen Eigenschaft eines Lebewesens, aufweisend:
- eine Detektoranordnung 10 mit einer Impedanzmesseinheit 12 und einer Elektrodenanordnung 11 zum Erfassen eines der thorakalen Eigenschaft zugeordneten Messsignals in Form eines Impedanzsignals;
- eine an der Detektoranordnung angeschlossene Überwachungsanordnung 20, die eingerichtet ist, einen Parameter aus dem Messsignal zu erzeugen, der indikativ für die thorakale Eigenschaft ist, und
- eine an der Überwachungsanordnung 20 angeschlossene Auswerteeinheit 30, die eingerichtet ist, ein Auswerteergebnis zur thorakalen Eigenschaft aus dem Parameter zu quantifizieren. Dabei ist vorgesehen, dass die Elektrodenanordnung 11 wenigstens eine auf dem Gehäuse angeordneten Elektrodenstruktur einer Anzahl von gegeneinander isolierten, über die Impedanzmesseinheit 12 wirkverbundene separat ansteuerbare Elektroden 11.1, 11.2, 11.3 aufweist, wobei ein Elektrodenkörper eine Streifenform hat.

### Bezugszeichenliste

- 1: Herz
- 2: Thorax
- 10: Detektoranordnung
- 11: Elektrodenanordnung
- 11 A: Elektrodenpaar
- 11B: Elektrodenpaar
- 11.1: Elektrode
- 11.2: Elektrode
- 11.3: Elektrode
- 11.4: Elektrode
- 11.5: Elektrode
- 11.6: Elektrode
- 12: Impedanzmesseinheit
- 20: Überwachungsanordnung
- 30: Auswerteeinheit
- 40: Kommunikationsmodul
- 50: Gehäuse
- 51: Header
- 52: Gehäusebehälter
- 53: Verankerung
- 54: Kabel
- 60: Mess- und Steuerelektronik
- 70: Telemetrieverbindung
- 100: elektromedizinisches Implantat
- 200: Empfangseinheit
- 300: Speichereinheit
- 1000: Überwachungssystem
- D: Detail
- F_{A}: Feldlinie
- F_{B}: analoge Feldlinie

## Patentansprüche

1. Elektromedizinisches Implantat (100) zum Überwachen einer thorakalen Eigenschaft eines Lebewesens, mit einem Gehäuse (50), aufweisend:
- eine Detektoranordnung (10) mit einer Impedanzmesseinheit (12) und einer Elektrodenanordnung (11), die eingerichtet sind zum Erfassen eines der thorakalen Eigenschaft zugeordneten Messsignals in Form eines Impedanzsignals;
- eine an der Detektoranordnung (10) angeschlossene Überwachungsanordnung (20), die eingerichtet ist, einen Parameter aus dem Messsignal zu erzeugen, der indikativ für die thorakale Eigenschaft ist, und
- eine an der Überwachungsanordnung (20) angeschlossene Auswerteeinheit (30), die eingerichtet ist, ein Auswerteergebnis zur thorakalen Eigenschaft aus dem Parameter zu bestimmen;
**dadurch gekennzeichnet, dass**
- die Elektrodenanordnung (11) wenigstens eine auf dem Gehäuse (50) angeordnete Anzahl von gegeneinander isolierten, über die Impedanzmesseinheit (12) wirkverbundene und separat ansteuerbare Elektroden (11.1, 11.2, 11.3) aufweist, wobei ein Elektrodenkörper eine Streifenform hat, und
- die Elektroden über die Impedanzmesseinheit (12) als paarweise zusammenwirkende Elektrodenpaare (11A, 11B) betreibbar sind, und
ein Abstand der Elektroden eines ersten Elektrodenpaares (11A) größer als ein Abstand der Elektroden eines zweiten Elektrodenpaares (11B) ist.

2. Elektromedizinisches Implantat (100) nach Anspruch 1, **dadurch gekennzeichnet, dass** die Impedanzmesseinheit (12) ein Erregermodul zum Abgeben eines elektrischen Stroms über die Elektrodenanordnung (11) und ein Tastmodul zum Annehmen einer Spannung über die Elektrodenanordnung (11) aufweist.

3. Elektromedizinisches Implantat (100) nach Anspruch 1 oder 2, weiter aufweisend ein Kommunikationsmodul (40) zur drahtlosen Übermittlung des Auswerteergebnisses in einem Auswertesignal.

4. Elektromedizinisches Implantat (100) nach einem der Ansprüche 1 bis 3, **dadurch gekennzeichnet, dass** die Elektrodenanordnung (11) wenigstens eine nahe zum Implantat angeordnete Elektrode (11.1, 11.2, 11.3) umfasst, und/oder eine entfernt zum Implantat (100) angeordnete zweite Elektrode als Teil einer Elektrodenstruktur aufwei st.

5. Elektromedizinisches Implantat (100) nach einem der Ansprüche 1 bis 4, **dadurch gekennzeichnet, dass** die Elektrodenanordnung (11) wenigstens eine entfernt zum Implantat angeordnete zweite Elektrode (11.4, 11.5, 11.6) umfasst, wobei die entfernt zum Implantat (100) angeordnete zweite Elektrode Teil einer weiteren Elektrodenstruktur ist.

6. Elektromedizinisches Implantat (100) nach einem der Ansprüche 1 bis 5, **dadurch gekennzeichnet, dass** eine erste Elektrodenstruktur der Elektrodenanordnung (11) auf dem Gehäuse (50) gebildet ist und/oder eine zweite Elektrodenstruktur der Elektrodenanordnung (11) auf einem am Gehäuse (50) angeschlossenen Elektrodenkabel (54) gebildet ist.

7. Elektromedizinisches Implantat (100) nach einem der Ansprüche 1 bis 6, **dadurch gekennzeichnet, dass** die Elektrodenstruktur einen Elektrodenkörper in Form einer Streifenelektrode, einer Ringelektrode oder einer Interdigitalelektrode gebildet ist.

8. Elektromedizinisches Implantat (100) nach einem der Ansprüche 1 bis 7, **dadurch gekennzeichnet, dass** eine erste Elektrode (11.1-11.6) und/oder ein erstes Elektrodenpaar (11A) der Elektrodenanordnung (11) separat mit einer von einer weiteren Elektrode (11.1-11.6) und/oder Elektrodenpaar (11B) unterschiedlichen elektrischen Frequenz und/oder Amplitude und/oder Phase ansteuerbar ist.

9. Elektromedizinisches Implantat (100) nach einem der Ansprüche 1 bis 8, **dadurch gekennzeichnet, dass** ein erstes Messsignal in Form eines ersten Impedanzsignals auf Basis einer ersten Erregerfrequenz gebildet ist, und/oder ein zweites Messsignal in Form eines zweiten Impedanzsignals auf Basis einer zweiten Erregerfrequenz gebildet ist.

10. Elektromedizinisches Implantat (100) nach einem der Ansprüche 1 bis 9, **dadurch gekennzeichnet, dass** die Elektrodenstruktur eine Anzahl von wenigstens drei Elektrodenkörpern (11.1-11.6) aufweist.

11. Elektromedizinisches Implantat (100) nach einem der Ansprüche 1 bis 10, **dadurch gekennzeichnet, dass** die Anzahl von Elektroden (11.1, 11.2, 11.3) symmetrisch zu einem Gehäusebehälter (52) des Implantats (100) angeordnet sind, und dabei mehrseitig oder allseitig oder ringförmig um einen Gehäusebehälter (52) des Implantats (100) angeordnet sind.

12. Elektromedizinisches Implantat (100) nach einem der Ansprüche 1 bis 11, **dadurch gekennzeichnet, dass** die Detektoranordnung zum Erfassen des ersten Messsignals umfasst:
eine EKG-Messeinheit mit einer Elektrodenanordnung (11), die eingerichtet sind das erste Messsignal in Form eines (Elektrokardiogramm-) EKG-Signals zu detektieren.

13. Elektromedizinisches Implantat (100) nach einem der Ansprüche 1 bis 12, **dadurch gekennzeichnet, dass** die Detektoranordnung zum Erfassen einer thorakalen Eigenschaft, einen oder mehrere der Detektoren umfasst, die ausgewählt sind aus der Gruppe bestehend aus:
akustischer Detektor, optischer Detektor, Druck- und/oder Dehnungs-Detektor, Beschleunigungsdetektor, Temperaturdetektor, Sauerstoffdetektor, biochemischer Detektor.

14. Überwachungssystem (1000) aufweisend das elektromedizinische Implantat (100) nach einem der vorhergehenden Ansprüche, das
- ein Kommunikationsmodul (40) zur drahtlosen Aussendung des Auswerteergebnisses in einem Auswertesignals vom Implantat (100) aufweist, weiter aufweisend:
- eine Empfangseinheit (200) zum Empfangen des Auswertesignals;
- eine Speichereinheit (300) für das Auswerteergebnis.

## Claims

1. An electromedical implant (100) for monitoring a thoracic property of a living being, the implant comprising a housing (50), having:
- a detector arrangement (10) comprising an impedance measuring unit (12) and an electrode arrangement (11), which are configured to capture a measurement signal associated with the thoracic property in the form of an impedance signal;
- a monitoring arrangement (20) connected to the detector arrangement (10) and configured to generate a parameter from the measurement signal that is indicative of the thoracic property, and
- an evaluation unit (30) connected to the monitoring arrangement (20) and configured to determine an evaluation result regarding the thoracic property from the parameter;
**characterised in that**
- the electrode arrangement (11) has at least one number of mutually isolated electrodes (11.1, 11.2, 11.3) that are disposed on the housing (50) and are operatively connected via the impedance measuring unit (12) and can be controlled separately, wherein an electrode body has the form of a strip, and
- the electrodes can be operated via the impedance measuring unit (12) as electrode pairs (11A, 11B) cooperating in pairs, and
a spacing between the electrodes of a first electrode pair (11A) is greater than a spacing between the electrodes of a second electrode pair (11B).

2. The electromedical implant (100) according to Claim 1, **characterised in that** the impedance measuring unit (12) has an exciter module for delivering an electric current via the electrode arrangement (11) and a sampling module configured to receive a voltage via the electrode arrangement (11).

3. The electromedical implant (100) according to Claim 1 or 2, further having a communication module (40) configured to wirelessly transmit the evaluation result in an evaluation signal.

4. The electromedical implant (100) according to one of Claims 1 to 3, **characterised in that** the electrode arrangement (11) comprises at least one electrode (11.1, 11.2, 11.3) disposed close to the implant, and/or has a second electrode disposed away from the implant (100) as part of an electrode structure.

5. The electromedical implant (100) according to one of Claims 1 to 4, **characterised in that** the electrode arrangement (11) comprises at least one second electrode (11.4, 11.5, 11.6) disposed away from the implant, wherein the second electrode disposed away from the implant (100) is part of a further electrode structure.

6. The electromedical implant (100) according to one of Claims 1 to 5, **characterised in that** a first electrode structure of the electrode arrangement (11) is formed on the housing (50) and/or a second electrode structure of the electrode arrangement (11) is formed on an electrode lead (54) connected to the housing (50).

7. The electromedical implant (100) according to one of Claims 1 to 6, **characterised in that** the electrode structure is formed by an electrode body in the form of a strip electrode, an annular electrode, or an interdigital electrode.

8. The electromedical implant (100) according to one of Claims 1 to 7, **characterised in that** a first electrode (11.1-11.6) and/or a first electrode pair (11A) of the electrode arrangement (11) is controllable separately using an electric frequency and/or amplitude and/or phase that is different from a further electrode (11.1-11.6) and/or electrode pair (11B).

9. The electromedical implant (100) according to one of Claims 1 to 8, **characterised in that** a first measurement signal in the form of a first impedance signal is formed on the basis of a first exciter frequency, and/or a second measurement signal in the form of a second impedance signal is formed on the basis of a second exciter frequency.

10. The electromedical implant (100) according to one of Claims 1 to 9, **characterised in that** the electrode structure comprises a number of at least three electrode bodies (11.1-11.6).

11. The electromedical implant (100) according to one of Claims 1 to 10, **characterised in that** the number of electrodes (11.1, 11.2, 11.3) are disposed symmetrically with respect to a housing container (52) of the implant (100), and are disposed on several sides of, or all sides of, or in an annular shape around a housing container (52) of the implant (100).

12. The electromedical implant (100) according to one of Claims 1 to 11, **characterised in that** the detector arrangement for capturing the first measurement signal comprises:
an ECG (electrocardiogram) measuring unit comprising an electrode arrangement (11), which is equipped to detect the first measurement signal in the form of an ECG signal.

13. The electromedical implant (100) according to one of Claims 1 to 12, **characterised in that** the detector arrangement for capturing a thoracic property comprises one or more detectors, selected from the group consisting of:
acoustic detectors, optical detectors, pressure and/or expansion detectors, acceleration detectors, temperature detectors, oxygen detectors, and/or biochemical detectors.

14. A monitoring system (1000) having the electromedical implant (100) according to one of the preceding claims, which has
- a communication module (40) configured to wirelessly transmit the evaluation result in an evaluation signal from the implant (100); and also having
- a receiving unit (200) configured to receive the evaluation signal; and
- a memory unit (300) for the evaluation result.

## Revendications

1. Implant électromédical (100) pour la surveillance d'une propriété thoracique dans un être vivant, avec un boîtier (50) présentant :
- un agencement de détecteurs (10) avec une unité d'impédance (12) et un agencement d'électrodes (11), qui sont installées pour la détection d'un signal de mesure associé à une propriété thoracique sous la forme d'un signal d'impédance ;
- un ensemble de surveillance (20), adjoint à l'agencement de détecteurs (10), qui est installé pour générer un paramètre à partir du signal de mesure qui est indicatif de la propriété thoracique, et
- une unité d'évaluation (30), adjointe à l'ensemble de surveillance (20), qui est installée pour déterminer un résultat d'évaluation pour la propriété thoracique à partir du paramètre ;
**caractérisé en ce que**
- l'agencement d'électrodes (11) présente au moins un nombre d'électrodes (11.1, 11.2, 11.3) isolées les unes par rapport aux autres, disposées sur le boîtier (50), reliées par l'unité d'impédance (12) et pouvant être mises en service séparément, où un corps d'électrode a une forme de bande, et
- les électrodes peuvent être mises en service sous la forme de paires d'électrodes (11A, 11B) agissant conjointement par l'intermédiaire de l'unité de mesure d'impédance (12), et
une distance des électrodes de la première paire d'électrodes (11A) est supérieure à une distance des électrodes d'une deuxième paire d'électrodes (11B).

2. Implant électromédical (100) selon la revendication 1, **caractérisé en ce que** l'unité de mesure d'impédance (12) présente un module d'excitation pour la délivrance d'un courant électrique par l'intermédiaire de l'agencement d'électrodes (11) et un module tactile pour la réception d'une tension par l'intermédiaire de l'agencement d'électrodes (11).

3. Implant électromédical (100) selon les revendications 1 ou 2, présentant en outre un module de communication (40) pour la transmission sans fil du résultat d'exploitation en un signal d'exploitation.

4. Implant électromédical (100) selon l'une des revendications 1 à 3, **caractérisé en ce que** l'agencement d'électrodes (11) comprend au moins une électrode (11.1, 11.2, 11.3) disposée près de l'implant, et/ou présente une deuxième électrode en tant que composante d'une structure d'électrodes éloignée par rapport à l'implant (100).

5. Implant électromédical (100) selon l'une des revendications 1 à 4, **caractérisé en ce que** l'agencement d'électrodes (11) comprend au moins une deuxième électrode (11.4, 11.5, 11.6) disposée éloignée par rapport à l'implant, où la deuxième électrode disposée éloignée par rapport à l'implant (100) est une composante d'une autre structure d'électrodes.

6. Implant électromédical (100) selon l'une des revendications 1 à 5, **caractérisé en ce que** la première structure d'électrodes de l'agencement d'électrodes (11) est formée sur le boitier (50) et/ou une deuxième structure d'électrodes de l'agencement d'électrodes (11) est montée sur un câble d'électrode (54) raccordé au boitier (50).

7. Implant électromédical (100) selon l'une des revendications 1 à 6, **caractérisé en ce que** la structure d'électrodes est formée par un corps d'électrode sous la forme d'une électrode en bande, d'une électrode annulaire ou d'une électrode interdigitale.

8. Implant électromédical (100) selon l'une des revendications 1 à 7, **caractérisé en ce qu'**une première électrode (11.1 à 11.6), et/ou qu'une première paire d'électrodes (11A) de l'agencement d'électrodes (11) peut être mise en service de manière séparée avec une fréquence, et/ou une amplitude, et/ou une phase électrique différente de celle d'une autre électrode (11.1 à 11.6), et/ou d'une autre paire d'électrodes (11B).

9. Implant électromédical (100) selon l'une des revendications 1 à 8, **caractérisé en ce qu'**un premier signal de mesure est créé sous la forme d'un premier signal d'impédance sur une base d'une première fréquence d'excitation, et/ou un deuxième signal de mesure est créé sous forme d'un deuxième signal d'impédance sur une base d'une deuxième fréquence d'excitation.

10. Implant électromédical (100) selon l'une des revendications 1 à 9, **caractérisé en ce que** la structure d'électrodes présente un nombre parmi au moins trois corps d'électrodes (11.1 à 11.6).

11. Implant électromédical (100) selon l'une des revendications 1 à 10, **caractérisé en ce que** le nombre d'électrodes (11.1, 11.2, 11.3) est disposé de manière symétrique par rapport à un emballage de boitier (52) de l'implant (100), et ainsi elles sont disposées sur plusieurs côtés, ou de tous les côtés, ou en forme d'anneau, autour d'un emballage de boitier (52) de l'implant (100).

12. Implant électromédical (100) selon l'une des revendications 1 à 11, **caractérisé en ce que** l'agencement de détecteurs, pour la détection du premier signal de mesure, comprend :
une unité de mesure d'ECG avec un agencement d'électrodes (11), qui sont installées pour détecter le premier signal de mesure sous la forme d'un signal (d'électrocardiogramme) ECG.

13. Implant électromédical (100) selon l'une des revendications 1 à 12, **caractérisé en ce que** l'agencement de détecteurs comprend, pour la détection d'une propriété thoracique, un ou plusieurs parmi les détecteurs qui sont choisis dans le groupe constitué :
d'un détecteur acoustique, d'un détecteur optique, d'un détecteur de pression et/ou de traction, d'un détecteur de vitesse, d'un détecteur de température, d'un détecteur d'oxygène, d'un détecteur biochimique.

14. Système de surveillance (1000) comportant l'implant électromédical (100) selon l'une des revendications précédentes qui présente
- un module de communication (40) pour l'émission sans fil du résultat d'exploitation en un signal d'exploitation de l'implant (100), présentant en outre :
- une unité de réception (200) pour la réception du signal d'exploitation ;
- une unité de mémorisation (300) pour le résultat d'exploitation.
